(19) Europäisches Patentamt / European Patent Office / Office européen des brevets

(11) **EP 3 698 721 A2**

(12) **EUROPEAN PATENT APPLICATION**
published in accordance with Art. 153(4) EPC

(43) Date of publication:
**26.08.2020 Bulletin 2020/35**

(51) Int Cl.:
*A61B 8/08* (2006.01)     *A61B 8/00* (2006.01)

(21) Application number: **18857074.1**

(22) Date of filing: **15.11.2018**

(86) International application number:
**PCT/CN2018/115524**

(87) International publication number:
**WO 2019/052584 (21.03.2019 Gazette 2019/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.09.2017 CN 201710842214**

(71) Applicant: **Nie, Xiliang**
**Hubei 430014 (CN)**

(72) Inventor: **Nie, Xiliang**
**Hubei 430014 (CN)**

(74) Representative: **Monzón de la Flor, Luis Miguel**
**Poeta Joan Maragall, 9**
**esc. Izq 3o Izq.**
**28020 Madrid (ES)**

Remarks:
A request for restoration of the right of priority under Rule 49ter.2 PCT is pending before the EPO as designated Office.

(54) **HUMAN TISSUE QUASI-ELASTIC COEFFICIENT AND ELASTICITY MEASUREMENT METHODS AND DEVICE**

(57)     A human tissue quasi-elastic coefficient measurement method, wherein a simplex optimization method is used to determine human tissue quasi-elastic coefficients, and nine elastic coefficients in three dimensions are reduced to three quasi-elastic coefficients (for two-dimensional ultrasound patient treatment planning or probing, four elastic coefficients are reduced to two quasi-elastic coefficients), thereby saving calculation time. Also provided is a human tissue quasi-elasticity measurement method based on the human tissue quasi-elastic coefficient measurement method, wherein a secondary imaging method is used to measure relative changes in human tissue quasi-elastic coefficients, thereby improving defects in elasticity imaging devices which are dependent on ultrasound imaging accuracy. Changes in human tissue structures may be accurately determined by measuring the relative changes. Also provided is an ultrasound probe or probing device matched with the human tissue quasi-elastic coefficient measurement, which obviates the need for expensive imaging devices, thereby lowering costs.

FIGURE 1

**Description**

**TECHNICAL FIELD**

The invention relates to the field of detection, in particular to a method and a device for determining the change of a human tissue quasi-elastic coefficient and a quasi-elastic coefficient. The invention also provides a patient treatment plan based on the ultrasonic image, on the basis of the plan, the patient data plan can be made every day, and the patient data plan can be accurately made with the change of the tumor size.

**BACKGROUND OF THE INVENTION**

At present, the absolute elastic coefficient of human tissue is determined through an elastic image instrument (such as Ge and Michael), but the elastic image instrument generally needs an imaging device which is expensive in price and high in precision, is not accepted by a doctor clinically and is not large in market amount.

According to the prior quasi-elastic coefficient measuring method, the three-dimensional nine elastic coefficients or four elastic coefficients of two dimensions are used for measuring and calculating, the calculation time is long, and because the absolute elasticity of the human tissue is a difficult determination, even if the absolute elastic coefficient can be obtained, the difference of the elastic coefficients of the normal tissue and the diseased tissue is not easily distinguished (such as the calcification of the human body or the density of the benign tumor and the tumor is very similar)

As can be seen, the above-described prior art elastic coefficient determination methods and apparatus are clearly still present with great inconvenience and drawbacks, without the need for a theoretical breakthrough to solve this problem.

SUMMARY OF **THE INVENTION**

The first technical problem to be solved by the invention is to provide a method for measuring the human tissue quasi-elastic coefficient, so that the calculation time is saved, and the defects of long calculation time of an existing elastic coefficient are overcome.

In order to solve the technical problem, the invention adopts the following technical scheme:

The invention provides a method for determining a human tissue quasi-elastic coefficient The method comprises the following steps: (1) determining a human tissue quasi-elastic coefficient by adopting a Simplex optimization method:

$$T = E_{xx}/\Delta x + E_{yy} \Delta y + E_{zz} \Delta z \qquad (1)$$

In the formula (1): T is the tension of human tissue, $E_{XX}$, $E_{YY}$ and $E_{ZZ}$ are three main human tissue elasticity coefficients, $\Delta X$, $\Delta Y$, and $\Delta z$ are the displacement after the human tissue adds pressure;

Specifically:

(1) The set of hypothetical elastic coefficients $E_{XX0}$, $E_{YY0}$, $E_{ZZ0}$ are first placed as input points, delta x, delta y, delta z are determined by the image density obtained by three-dimensional ultrasound (or two-dimensional) detection, and a tension is calculated;
(2) The $E_{XX}$, $E_{YY}$ and $E_{ZZ}$ are adjusted according to the Simplex optimization method, and a new tension is calculated;
(3) Continuing until $T_n-T_{n-1}$ tends to zero, a value of the human tissue quasi-elastic coefficient is obtained.

$s a further refinement, the smallest division unit of the detection region of the three-dimensional ultrasound (or two-dimensional) image is 1 nm or 1 nm or 1 nm (for two-dimensional 1 nm or 1 nm) or lower.

The invention further provides a method for determining the human tissue quasi-elastic coefficient, and the elasticity coefficient of the human tissue is determined based on the following formula (2) by adopting a Simplex optimization method:

$$T = E_{xx}/\Delta x + E_{yy} \Delta y \qquad (2)$$

In the formula (2), T is the tension of the human tissue, $E_{xx}$ and $E_{YY}$ are two main human tissue elasticity coefficients, $\Delta X$ and $\Delta y$ are the displacement after the human tissue adds pressure;

Specifically:

(1) a set of hypothetical elastic coefficients EXX0, EYY0 are first placed as input points, delta x, delta y are determined by the image density obtained by two-dimensional ultrasonic detection, and a tension is calculated;

(2) According to the Simplex optimization method, $E_{XX}$ and $E_{YY}$ are adjusted, and a new tension is obtained;

(3) Continuing until $T_n - T_{n-1}$ tends to zero, a value of the human tissue quasi-elastic coefficient is obtained.

As a further refinement, the smallest division unit of the detection region of the two-dimensional ultrasound image is 1 nm or 1 nm or less.

In addition, the invention also provides a method for measuring the human tissue quasi-elasticity, so that the accuracy dependence of the ultrasonic image is completely separated, so that the design surpasses the physical limit of the ultrasonic image instrument.. Moreover, the change value of the quasi-elastic coefficient obtained according to the secondary imaging method can accurately distinguish normal tissues and diseased tissues of the human body, thereby overcoming the uncertainty of the existing absolute elastic coefficient determination.

In order to solve the technical problem, the invention adopts the following technical scheme:
The invention discloses a method for measuring tissue elasticity of a human body, which adopts a secondary imaging method to determine the relative change condition of a human tissue quasi-elastic coefficient, and the determination of the tissue quasi-elastic coefficient of each human body adopts the human tissue quasi-elastic coefficient determination method

The invention further provides a special ultrasonic detector in the method for determining the human tissue quasi-elastic coefficient, so that the ultrasonic detector does not need to rely on an expensive ultrasonic image instrument.

In order to solve the technical problem, the invention adopts the following technical scheme:
The invention discloses a special ultrasonic detector in a method for measuring the human tissue quasi-elastic coefficient, wherein the ultrasonic detector comprises an ultrasonic sensor, and an output port of the ultrasonic sensor is in the form of a USB output port

The invention further provides an ultrasonic detection device, so that the calculation time is short, the cost is low, and the ultrasonic detection device is easy to popularize and use.

In order to solve the technical problem, the invention adopts the following technical scheme:
An ultrasonic detection apparatus, comprising:

The ultrasonic sensor is suitable for obtaining the tissue image density and transmitting the tissue image density to the processor through an output port;

A brand-new change image software for analyzing the quasi-elastic coefficient of human tissues to accurately distinguish normal cells and tumor cells, and the distinguishing precision can reach even more than an existing most advanced tumor detector, so that the physical limit of the existing most advanced tumor detector is overcome;

The human tissue quasi-elastic coefficient was determined based on the following equation (1) using a Simplex optimization method:

$$T = E_{xx}/\Delta x + E_{yy} \Delta y + E_{zz} \Delta z \qquad (1)$$

In the formula (1): T is the tension of human tissue, $E_{XX}$, $E_{YY}$ and $E_{ZZ}$ are three main human tissue quasi-elastic coefficients, $\Delta X$, $\Delta Y$ and $\Delta z$ are the displacement after the human tissue adds pressure;

A set of hypothetical quasi-elastic coefficients $E_{XX0}$, $E_{YY0}$, $E_{ZZ0}$ are first placed as input points, delta x, delta y, delta z are determined by the image density obtained by three-dimensional ultrasound (or two-dimensional) detection, and a tension is calculated;

The $E_{XX}$, $E_{YY}$, and $E_{ZZ}$ were adjusted according to the Simplex optimization method and a new tension was calculated;

Continuing until $T_n - T_{n-1}$ tends to be zero, a value of the human tissue quasi-elastic coefficient is obtained.

The invention further provides an ultrasonic detection device which comprises the following components:

The ultrasonic sensor is suitable for obtaining the tissue image density and transmitting the tissue image density to the processor through an output port;

A brand-new change image software for analyzing the quasi-elastic coefficient of human tissues to accurately distinguish normal cells and tumor cells, and the distinguishing precision can reach even more than an existing most advanced tumor detector, so that the physical limit of the existing most advanced tumor detector is overcome;

The human tissue quasi-elastic coefficient was determined based on the following equation (2) using a Simplex optimization method:

$$T = E_{xx}/\Delta x + E_{yy}\,\Delta y \qquad\qquad (2)$$

In the formula (2), T is the tension of the human tissue, $E_{XX}$ and $E_{YY}$ are two main human tissue elasticity coefficients, $\Delta X$ and $\Delta y$ are the displacement after the human tissue adds pressure;

A set of hypothetical quasi-elastic coefficients $E_{XX0}$, $E_{YY0}$ are first placed as input points, delta x, delta y are determined by the image density obtained by two-dimensional ultrasonic detection, and a tension is calculated;

The $E_{XX}$, $E_{YY}$ is adjusted according to the Simplex optimization method, and a new tension is obtained;

Continuing until $T_n - T_{n-1}$ tends to be zero, a value of the tissue elasticity coefficient is obtained.

As a further improvement, the output port of the ultrasonic sensor is in the form of a USB output port.

By employing the above technical solution, the present invention has at least the following advantages:

(1) The invention introduces a new way of simplex optimization to determine human tissue quasi-elastic coefficients, and reduces the nine elastic coefficients of the three-dimensional stereo to three elastic coefficients (quasi-elastic coefficients), or reduces the four elastic coefficients in two dimensions to two elastic coefficients, saving computation time.

(2) According to the invention, the time-varying quasi-elastic coefficient (relative quasi-elastic coefficient) is introduced for the first time to determine the change condition of the human tissue quasi-elastic coefficient, and the defect of an elastic image instrument relying on the accuracy of the ultrasonic image is thoroughly changed; the detection precision can be 1 nm or 1 nm or 1 nm (for two-dimensional 1 nm or 1 nm), and even smaller units are 0.1 nm or 0.1 nm (for two-dimensional 0.1 nm or 0.1 nm).

(3) The relative elastic coefficient is adopted, and the accuracy of the image is low relative to the absolute elastic coefficient, so that the quasi-elastic coefficient obtained at a time is not the real elastic coefficient of the measured part, and the precision of the method is not affected, since the error of the measured elastic coefficient is counteracted in the two measurements and the defect of the data error of the absolute elastic coefficient measurement is avoided; In addition, the relative quasi-elastic coefficient is adopted, the change condition of the elastic structure of the human tissue can also be found relative to the absolute elastic coefficient, accurate guidance is provided for distinguishing normal tissues and diseased tissues, and the method can detect the just-occurring condition of the tumor, so that the design patent can detect the condition that the tumor has just taken place.

(4) The ultrasonic detector and the ultrasonic detection equipment of the invention reduce the traditional expensive imaging equipment, and only one data output port is needed for data output, so that the market price can be popularized to the degree that a single household is acceptable, and the detection of the general human tissue quasi-elastic coefficient can be accurately self-measured at home.

BRIEF DESCRIPTION OF THE DRAWINGS

So that the manner in which the above recited features of the present invention can be understood in detail, a more particular description of the invention, briefly summarized above, may be had by reference to the embodiments thereof which are illustrated in the appended drawings

FIG. 1 is a schematic view of an ultrasonic probe of the present invention;
FIG. 2 is a structural block diagram of an ultrasonic detection device of the present invention;
FIG. 3 is a schematic representation of the detection accuracy of the present invention.

DETAILED DESCRIPTION OF THE PREFERRED EMBODIMENTS

Example 1. Determination of Human Tissue Quasi-Elastic Coefficients

Taking human tissue as an example, the embodiment of the invention introduces a new way in the determination of the human tissue quasi-elastic coefficient, namely, a Simplex optimization method is adopted to obtain the human tissue quasi-elastic coefficient. The method for three-dimensional ultrasound (or two-dimensional) detection is based on the following equation (1):

$$T = E_{xx}/\Delta x + E_{yy}\,\Delta y + E_{zz}\,\Delta z \qquad\qquad (1)$$

In the formula (1): T is the tension of human tissue, $E_{XX}$, $E_{YY}$ and $E_{ZZ}$ are three main human tissue elasticity coefficients, $\Delta X$, $\Delta Y$, and $\Delta z$ are the displacement after the human tissue adds pressure;

1. A set of hypothetical elastic coefficients $E_{XX0}$, $E_{YY0}$, $E_{ZZ0}$ are first placed as input points, delta X, delta Y, delta Z are determined by the image density obtained by three-dimensional ultrasound detection (specific details belong to common knowledge in ultrasound image recognition techniques, omitted here), and a tension is calculated;
2. The $E_{XX}$, $E_{YY}$, and $E_{ZZ}$ were adjusted according to the Simplex optimization method and a new tension was calculated;
3. Continuing with the above method until $T_n - T_{n-1}$ tends to be zero, ie, the last two tensions are nearly equal, the value of the quasi-elastic coefficient of human tissue is obtained.

The initial value of the quasi-elastic coefficient described above may start from 6 GPa.

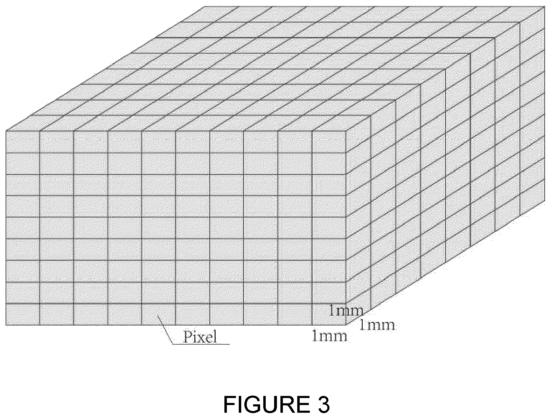

The accuracy of the above method is determined by the size of the human tissue detection unit, which in this example is set to 1 NMX 1 NMX 1 nm (for two-dimensional 1 NMX 1 nm), which is described in detail in FIG. 3, which is the accuracy of this embodiment. This accuracy is now the accuracy of CT, MRI, PET. The method of the invention can even improve the accuracy by 0.1 NMX/0.1 NMX/0.1 nm (for two-dimensional 0.1 NMX/0.1 nm), but at this point the calculation time can be long and clinically not allowed, but the method has the potential of improving the accuracy.

For two-dimensional ultrasonic planned detection, the tissue elasticity coefficient is determined based on the following equation (2) using a Simplex optimization method:

$$T = E_{xx}/\Delta x + E_{yy}\,\Delta y \qquad\qquad (2)$$

In the formula (2), T is the tension of the human tissue, EXX and $E_{YY}$ are two main human tissue quasi-elastic coefficients, $\Delta X$ and $\Delta y$ are the displacement after the human tissue adds pressure;
A set of hypothetical elastic coefficients $E_{XX0}$, $E_{YY0}$ are first placed as input points, delta x, delta y are determined by the image density obtained by two-dimensional ultrasonic detection, and a tension is calculated;
The $E_{XX}$, $E_{YY}$ is adjusted according to the Simplex optimization method, and a new tension is obtained;
Continuing until $T_n - T_{n-1}$ tends to be zero, a value of the tissue elasticity coefficient is obtained.

Example 2. Method for Determining Human Tissue Quasi-Elasticity

Aiming at human tissue quasi-elastic determination, the method mainly adopts a secondary imaging method to determine the relative change condition of the human tissue quasi-elastic coefficient, and the determination of the tissue quasi-elastic coefficient of each human body adopts the determination method in Example 1. A human tissue quasi-elastic coefficient is determined by first ultrasonic imaging, then another human body tissue quasi-elastic coefficient is determined through second ultrasonic imaging for a period of time, and the normal cell and the tumor cell can be accurately distinguished through the change of the human tissue quasi-elastic coefficient compared with the change of the human tissue quasi-elastic coefficient twice.

For a tumor patient, the patient can be separated for one week (the time can be self-adjusted according to the situation) by using the determination method of the human tissue quasi-elastic coefficient, and if we can make the ultrasound image every day in the treatment plan of the patient to determine the change of the tumor in the treatment, so that the damage to the normal cell can be reduced; if the quasi-elastic coefficients measured twice are changed, the change is a tumor cell. Since the absolute elastic coefficient distinguishes the tumor tissue and the normal cell tissue from the numerical size of the elastic coefficient, it is difficult to judge the tumor or not the tumor with a numerical value, but the relative quasi-elastic coefficient is different, since the tumor tissue growth is fast, if the quasi-elastic coefficient in the same place is measured at a certain time interval, the tumor part can change, but the normal tissue (such as calcification and benign tumor) does not change.

It is particularly pointed out in the present invention that, Since changes in the elasticity coefficient of the human tissue are related to a number of factors, such as adherence to exercise, the elastic coefficients of the muscle tissue may vary, such as in a tumor, such as a heart disease or other similar disease, the elastic coefficient of the corresponding tissue may also vary, ie, for a person, a change in the quasi-elastic coefficient may be made using the quasi-elastic determination method of the present invention, but the result of the determination does not necessarily mean that there is a certain disease, or certain disease is present. This variation, however, may be indicative of the onset of a certain lesion structure and the above-described method of the present invention has an accurate guidance meaning.

Example 3. An Ultrasonic Probe

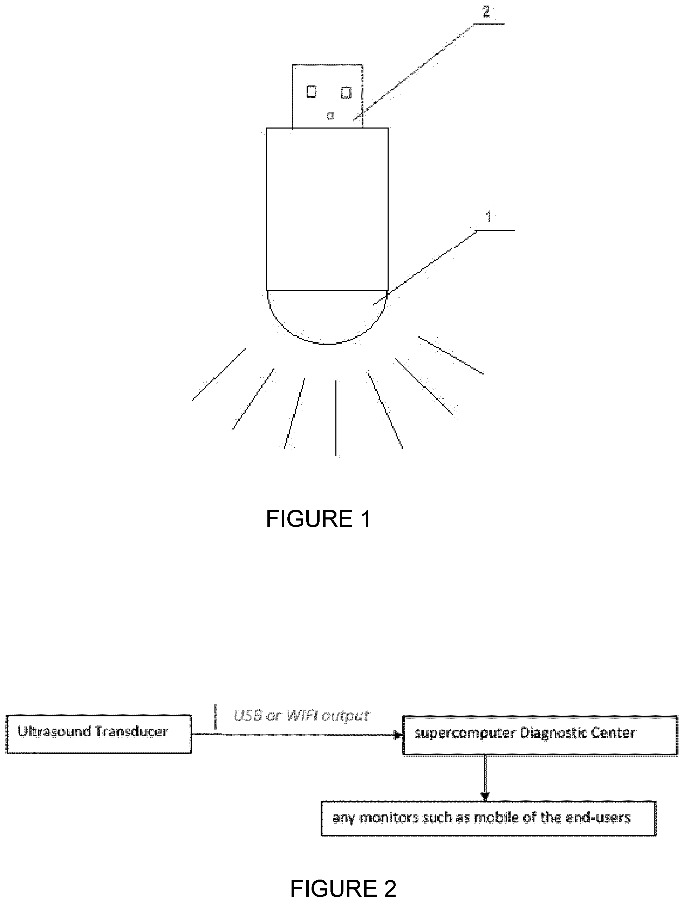

The embodiment of the invention provides a special ultrasonic detector in the method for determining the human tissue quasi-elastic coefficient, so that the ultrasonic detector does not need to rely on an expensive ultrasonic image instrument. As shown in FIG. 1, the ultrasonic probe comprises an ultrasonic sensor 1, and an output port 2 of the ultrasonic sensor 1 is in the form of a USB output port

Example 4. An Ultrasonic Detection Device

As shown in FIG. 2, the present embodiment provides an ultrasonic detection apparatus for use in the quasi-elastic coefficient determination method described above. For three-dimensional ultrasonic detection, the apparatus comprises:

The ultrasonic sensor is suitable for obtaining the tissue image density and transmitting the tissue image density to the processor through an output port;
A brand-new change image software for analyzing the quasi-elastic coefficient of human tissues to accurately distinguish normal cells and tumor cells, and the distinguishing precision can reach even more than an existing most advanced tumor detector, so that the physical limit of the existing most advanced tumor detector is overcome;
The human tissue elasticity coefficient was determined based on the following equation (1) using a Simplex optimization method:

$$T = E_{xx}/\Delta x + E_{yy} \, \Delta y + E_{zz} \, \Delta z \ (1)$$

In the formula (1): T is the tension of human tissue, $E_X$, $E_{YY}$ and $E_{ZZ}$ are three main human tissue elasticity coefficients, $\Delta X$, $\Delta Y$, and $\Delta z$ are the displacement after the human tissue adds pressure;

1. A set of hypothetical elastic coefficients $E_{XX0}$, $E_{YY0}$, $E_{ZZ0}$ are first placed as input points, delta X, delta Y, delta Z are determined by the image density obtained by three-dimensional ultrasonic detection, and a tension is calculated;

2. The $E_{xx}$, $E_{YY}$, and $E_{zz}$ were adjusted according to the Simplex optimization method and a new tension was calculated;

3. Continuing until $T_n - T_{n-1}$ tends to be zero, a value of the human tissue elasticity coefficient is obtained.

For two-dimensional ultrasound planned detection, the device comprises:

The ultrasonic sensor is suitable for obtaining the tissue image density and transmitting the tissue image density to the processor through an output port;
A brand-new change image software for analyzing the quasi-elastic coefficient of human tissues to accurately distinguish normal cells and tumor cells, and the distinguishing precision can reach even more than an existing most advanced tumor detector, so that the physical limit of the existing most advanced tumor detector is overcome;
The tissue elasticity coefficient was determined based on the following equation (2) using a Simplex optimization method:

$$T = E_{xx}/\Delta x + E_{yy} \, \Delta y \ (2)$$

In the formula (2), T is the tension of the human tissue, $E_{xx}$ and $E_{YY}$ are two main human tissue elasticity coefficients, $\Delta X$ and $\Delta y$ are the displacement after the human tissue adds pressure;
A set of hypothetical elastic coefficients $E_{XX0}$, $E_{YY0}$ are first placed as input points, delta x, delta y are determined by the image density obtained by two-dimensional ultrasonic detection, and a tension is calculated;
The $E_{XX}$, $E_{YY}$ is adjusted according to the Simplex optimization method, and a new tension is obtained;
Continuing until $T_n - T_{n-1}$ tends to be zero, a value of the human tissue elasticity coefficient is obtained.

Since the output port of the ultrasonic sensor does not need to be connected with the imaging device, the output port of the ultrasonic sensor can be in the form of a USB output port.

Similar to the conventional method, in the determination of each quasi-elastic coefficient, the ultrasonic detection device needs to take two measurements, the first pure ultrasonic detection device, the second time the water bag (or any method capable of generating pressure) is installed on the ultrasonic detection device, so that the pressure of the volume of the detection device is increased. In this way, the displacement of the tissue can be measured according to the density of the ultrasonic waves. The specific calculation time for each point of Simplex is about ten seconds. The specific procedure is described previously.

In summary, according to the method for determining the human tissue quasi-elastic coefficient, a simplex optimization method is adopted to determine the human tissue quasi-elastic coefficient, and the three-dimensional nine elastic coefficients are reduced to three elastic coefficients (quasi-elastic coefficients)) Or the four elastic coefficients of the two-dimensional are reduced to two elastic coefficients (quasi-elastic coefficients), so that the calculation time is saved; based on the quasi-elastic coefficient determination method, the relative change condition of the human tissue quasi-elastic coefficient is determined by adopting a secondary imaging method, the defect of an elastic image instrument relying on the ultrasonic image precision is thoroughly changed, and the precise guidance significance for the disease can be realized by determining the relative change condition; and in addition, The ultrasonic detector or the detection device matched with the human tissue quasi-elastic coefficient determination device does not need expensive imaging equipment, so that the price is low, the public acceptance degree is high, and the method is suitable for popularization and application.

Those skilled in the art will readily observe that numerous modifications, equivalents, and modifications may be made thereto without departing from the scope of the invention as defined by the appended claims.

**Claims**

1. The invention relates to a method for determining a human tissue quasi-elastic coefficient, which is a basic principle of the patent protection, and is **characterized in that** the method is based on the following formula (1) The human tissue quasi-elastic coefficient is determined by using a Simplex optimization method:

$$T = E_{XX} \Delta X + E_{YY} \Delta Y + E_{ZZ}/\Delta z \qquad (1)$$

wherein T is the tension of human tissue, $E_{XX}$, $E_{YY}$ and $E_{ZZ}$ are three main human tissue elasticity coefficients, namely, the quasi-elastic coefficient, $\Delta X$, $\Delta Y$ and $\Delta z$ in the patent are the displacement after the human tissue is subjected to pressure;
in particular,
The method comprises the following steps:

(1) placing a set of hypothetical elastic coefficients $E_{XX0}$, $E_{YY0}$, and $E_{ZZ0}$ as input points, and calculating to obtain a tension;
(2) adjusting $E_{XX}$, $E_{YY}$ and $E_{ZZ}$ by a simplex optimization method, and calculating to obtain a new tension;
(3) continuing to do until $T_n$-$T_{n-1}$ tends to be zero, thereby obtaining the value of the human tissue quasi-elastic coefficient.

2. The method of claim 1, wherein the minimum division unit of the detection region of the three-dimensional ultrasound image is 1 nm to 1 nm to nm.

3. A method of determining human tissue quasi-elastic coefficients, if treated with ultrasound images as a patient, is a patient treatment plan protected by this patent, all of which are protected by this patent in accordance with the principles of the present patent principles, **characterized by** the following formula: (2) The human tissue quasi-elastic coefficient is determined by using a Simplex optimization method:

$$T = E_{XX} \Delta X + E_{yy} \Delta y \qquad (2),$$

wherein T is the tension of human tissue, $E_{XX}$ and $E_{YY}$ are two main human tissue elasticity coefficients, $\Delta X$ and $\Delta y$ are the displacement after the human tissue adds pressure; in particular,
The method comprises the following steps:

(1) firstly, placing a set of assumed elastic coefficients $E_{xx0}$ and $E_{yy0}$ as input points, wherein $\Delta x$ and $\Delta y$ are determined according to the image density obtained by two-dimensional ultrasonic detection, and calculating to obtain a tension;

(2) adjusting $E_{xx}$ and $E_{yy}$ according to a Simplex optimization method to obtain a new tension;

(3) continuing to do until the $T_n$-$T_{n-1}$ tends to be zero, thereby obtaining the value of the human tissue quasi-elastic coefficient.

4. The method of claim 3, wherein the minimum division unit of the detection region of the two-dimensional ultrasound image is 1 nm to 1 nm.

5. The invention discloses a method for measuring tissue quasi-elasticity of a human body, which is **characterized in that** a secondary imaging method is used for determining the relative change condition of human tissue quasi-elastic coefficients, all the methods of using the secondary imaging method of the invention to perform pathological detection of a patient belong to the scope of the patent protection, and the method for measuring the tissue quasi-elasticity coefficient of the human body is adopted for determining the tissue quasi-elastic coefficient of each human body;

6. The special ultrasonic detector in the measuring method of the human tissue quasi-elastic coefficient according to any one of claims 1-4, wherein the ultrasonic detector comprises an ultrasonic sensor, the output port of the ultrasonic sensor is in the form of a USB output port, and all the production based on the ultrasonic detector of the present patent belongs to the protection category of the patent.

7. The ultrasonic detection device is **characterized by** comprising
an ultrasonic sensor which is suitable for obtaining tissue image density and transmitting the tissue image density to a processor;
a set of brand-new change image software for analyzing the quasi-elastic coefficient of human tissue to accurately distinguish normal cells and tumor cells, and the distinguishing precision can reach even more than an existing most advanced tumor detector, so that the physical limit of the existing most advanced tumor detector is overcome;
and the ultrasonic detection device is based on the following formula (1) The human tissue quasi-elastic coefficient is determined by using a Simplex optimization method:

$$T = E_{xx}\,\Delta x + E_{yy}\,\Delta y + E_{zz}/\Delta z \qquad (1)$$

in the formula (1): T is the tension of human tissue $E_{XX}$, $E_{YY}$, $E_{ZZ}$ are three major human tissue elasticity coefficients, $\Delta X$, $\Delta Y$, $\Delta Z$ are displacements after the body tissue has been subjected to pressure;
A set of hypothetical elastic coefficients $E_{xx0}$, $E_{yy0}$, $E_{zz0}$ are first placed as input points, $\Delta x$, $\Delta y$, $\Delta z$ are determined by
the image density obtained by three-dimensional ultrasonic detection, and a tension is obtained;
$E_{xx}$, $E_{yy}$, and $E_{zz}$ are adjusted according to the Simplex optimization method, and a new tension is obtained;
and continuing until $T_n$-$T_{n-1}$ tends to be zero The value of the human tissue quasi-elastic coefficient is obtained.

8. The ultrasonic detection device is **characterized by** comprising an ultrasonic sensor which is suitable for obtaining tissue image density and transmitting the tissue image density to a processor; a set of brand-new change image software for analyzing the quasi-elastic coefficient of human tissue to accurately distinguish normal cells and tumor cells, and the distinguishing precision can reach even more than an existing most advanced tumor detector, so that the physical limit of the existing most advanced tumor detector is overcome; and the ultrasonic detection device is based on the following formula (2) The human tissue quasi-elastic coefficient is determined by using a Simplex optimization method:

$$T = E_{xx}\,\Delta x + E_{yy}\,\Delta y \qquad (2)$$

Formula (2): T is the tension of human tissue, and $E_{XX}$ $E_{yy}$ is two major human tissue elasticity coefficients, $\Delta x$, $\Delta y$ is the displacement after the human tissue has been subjected to pressure;
A set of hypothetical elastic coefficients $E_{XX0}$, $E_{YY0}$ are first placed as input points, $\Delta X$, $AY$ are determined by the image density obtained by two-dimensional ultrasonic detection, and a tension is obtained; $E_{xx}$ and $E_{YY}$ are adjusted according to a Simplex optimization method, and a new tension is obtained;

continuing to do until $T_N$-$T_{N-1}$ tends to be zero, the value of the human tissue quasi-elastic coefficient is obtained.

9. The ultrasonic detection device of claim 7 or 8, wherein the output port of the ultrasonic sensor is in the form of a USB output port.

10. The patient according to claim 1-4 is also within the scope of this patent depending on the treatment plan of the ultrasound image.

FIGURE 1

FIGURE 2

**FIGURE 3**